# FASCICULE DE BREVET EUROPEEN

(11) **EP 2 490 626 B1**
(45) Date de publication et mention de la délivrance du brevet: **17.06.2015**
(21) Numéro de dépôt: 10785134.7
(22) Date de dépôt: 19.10.2010
(51) Int. Cl.: A61F 2/38, A61F 2/00

(54) **PROTHESE TOTALE DU GENOU A PLATEAU MOBILE**
KOMPLETTE KNIEPROTHESE MIT EINER MOBILEN PLATTE
TOTAL KNEE PROSTHESIS HAVING A MOBILE PLATE

(30) Priorité: 21.10.2009 FR 0957378
(43) Date de publication de la demande: 29.08.2012
(73) Titulaire: Buisson, Laurent, 73000 Chambery le Vieux (FR); Canovas, François, 34980 Montferrier sur Lez (FR); Farizon, Frédéric, 42400 Saint Chamond (FR); Mandrino, Alain, 06100 Nice (FR); Reynaud, Patrick, 69004 Lyon (FR); Aston Medical Developments Ltd, Londres NW1 1JD (GB)
(72) Inventeur: ALEPEE, Christophe, F-69003 Lyon (FR); MOATI, Jean-Claude, F-92130 Issy Les Moulineaux (FR); BUISSON, Laurent, F-73000 Chambery le Vieux (FR); CANOVAS, Francois, F-34980 Montferrier sur Lez (FR); FARIZON, Frédéric, F-42400 Saint Chamond (FR); MANDRINO, Alain, F-06100 Nice (FR); REYNAUD, Patrick, F-69004 Lyon (FR)
(74) Mandataire: Thivillier, Patrick
(86) Numéro de dépôt international: PCT/FR2010/052214
(87) Numéro de publication internationale: WO 2011/048315

(56) Documents cités:
- EP-A1- 0 970 667
- US-A- 5 906 643
- US-A1- 2003 009 229
- US-A1- 2003 009 232

## Description

L'invention se rattache au secteur technique des implants orthopédiques.

Plus particulièrement, l'invention concerne une prothèse du genou, notamment du type à glissement.

D'une manière parfaitement connue pour un homme du métier, ce type de prothèse comprend un composant fémoral monté avec capacité de flexion sur un composant tibial. Le composant fémoral présente généralement une trochlée prolongée, de part et d'autre d'une échancrure, par deux patins condyliens. Ces patins condyliens sont destinés à coopérer en appui, et généralement à glissement, sur un plateau tibial, le plus souvent réalisé en polyéthylène, et monté sur une embase tibiale, soit de manière fixe, soit avec capacité de déplacement.

Il est également connu, en fonction de la qualité des ligaments croisés du genou, d'utiliser soit une prothèse dite à conservation des croisés, soit une prothèse postéro-stabilisée au moyen généralement d'une came coopérant avec un plot central lié à l'embase tibiale pour remplacer le rôle des croisés absents. Le plot de stabilisation vient en butée avec la came, pour éviter tout phénomène de luxation antérieure des condyles en mouvement de flexion maximum.

Une solution de ce type ressort, par exemple, de l'enseignement du brevet EP 0 970 667 qui concerne une prothèse articulaire rotative et translatable avec stabilisation postérieure.

L'état de la technique le plus proche est consideré comme le document US 5906643A, qui définit le préambule de la revendication 1.

A partir de cet état de la technique, le problème que se propose de résoudre l'invention est d'améliorer la cinématique des composants fémoral et tibial, et de remédier à la limitation de flexion et diminuer l'usure du polyéthylène du plateau résultant des mouvements relatifs de translation des patins condyliens sur le plateau qui créent un effet de délamination de la couche supérieure du polyéthylène suite à ce frottement intense avec lesdits patins condyliens réalisés en métal.

Pour remédier à ces inconvénients, le problème que se propose de résoudre l'invention est de dissocier le mouvement de translation du plateau mobile entre l'embase et les patins condyliens lors de la flexion maximum et le mouvement congruent des patins condyliens et du plateau mobile, selon une flexion de 0 à 30° environ, correspondant à la marche. Le but recherché est donc de concevoir deux prothèses en une, c'est-à-dire un condyle congruent avec le plateau à cuvettes profondes dans le cycle de la marche, puis mobile et stabilisé lors de la flexion maximum.

Pour résoudre un tel problème, il a été conçu et mis au point une prothèse totale du genou à plateau mobile du type de celle comprenant, d'une manière connue, un composant fémoral constitué d'une trochlée prolongée, de part et d'autre d'une échancrure, par deux patins condyliens coopérant, avec capacité de flexion et de rotation, avec le plateau mobile monté avec capacité de déplacement antéropostérieur limité par rapport à une embase que présente un composant tibial.

L'invention est définie dans la revendication 1.

Selon l'invention, compte tenu du problème posé à résoudre, le composant fémoral présente un élément profilé en came disposé transversalement entre les deux patins condyliens, au niveau de l'extrémité libre de l'échancrure intercondylienne, et apte à coopérer en position de flexion à partir d'un angle d'environ 75° avec une zone d'appui profilée d'une manière correspondante que présente un plot rendu solidaire de ladite embase, de manière à provoquer, d'une manière concomitante, le déplacement antéropostérieur du plateau par rapport à l'embase, puis le recul limité dudit plateau en position de flexion maximum du composant fémoral par rapport au composant tibial.

Le plateau mobile étant libre en translation et en rotation, peut, par conséquent, avancer par rapport à l'embase tibiale en position d'extension maximum, pivoter au début de la flexion correspondant au mouvement naturel du fémur par rapport au tibia, puis reculer pour obtenir la flexion maximum nécessaire dans certains mouvements physiologiques.

Suivant une autre caractéristique, le plot déborde de l'embase au travers d'une ouverture du plateau conformée pour permettre audit plateau les mouvements de rotation et de déplacement antéropostérieur, ledit plot étant engagé dans un axe pivot asymétrique que présente l'embase.

Le plateau mobile, en polyéthylène, présente symétriquement deux cupules congruentes avec les patins condyliens en position d'extension suivant le cycle de marche, puis en position de flexion maximum. Le plot est en polyéthylène et est rendu solidaire de l'embase par une vis engagée dans une quille que présente ladite embase.

Pour résoudre le problème posé de permettre le déplacement du plateau par rapport à l'embase lors des mouvements de flexion, l'embase tibiale est plane et métallique et présente une surface polie d'appui et de glissement.

Avantageusement, le déplacement antéropostérieur du plateau par rapport à l'embase s'effectue, sous l'effet de la coopération du profilé en came avec le plot, depuis un angle de flexion d'environ 75° par rapport à l'axe du fémur jusqu'à un angle d'environ 125°.

L'invention est exposée ci-après plus en détail à l'aide des figures des dessins annexés dans lesquels :

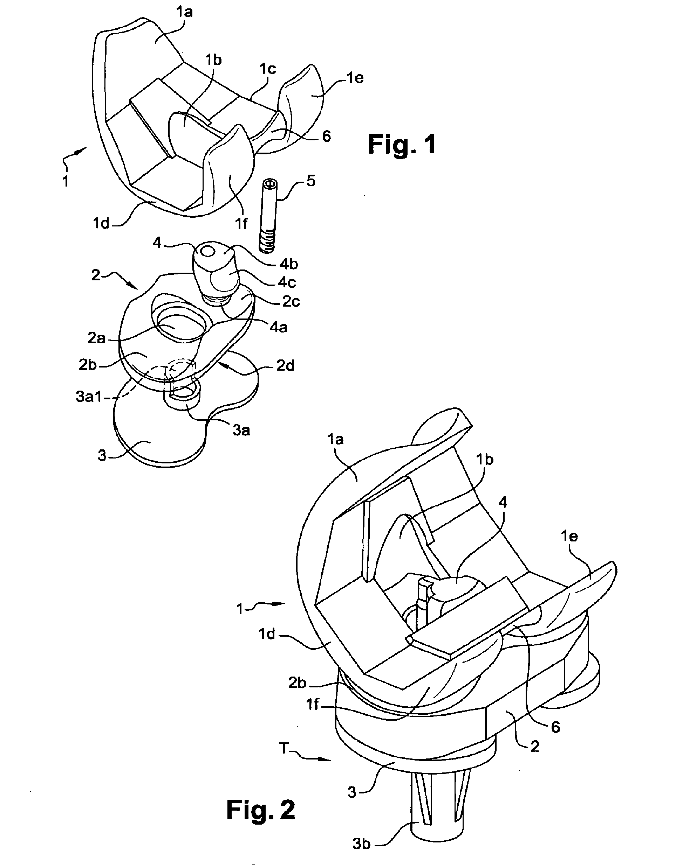

- la figure 1 est une vue en perspective avant montage des principaux éléments constitutifs de la prothèse selon l'invention ;
- la figure 2 est une vue en perspective correspondant à la figure 1 après montage et coopération des différents composants de la prothèse ;

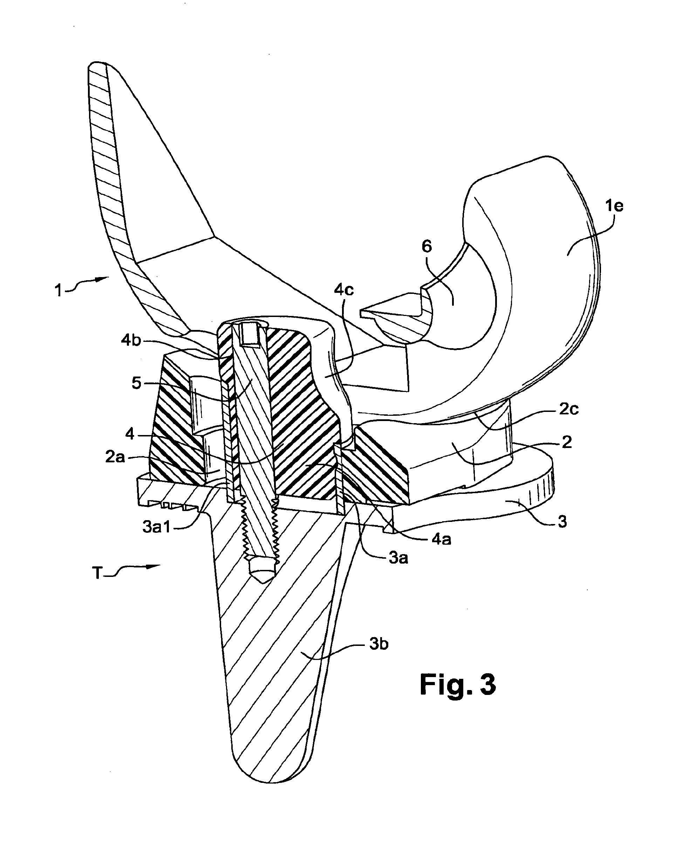

- la figure 3 est une vue en perspective en coupe longitudinale correspondant à la figure 2 ;
- les figures 4, 5, 6, 7, 8 et 9 sont des vues de côté à caractère schématique montrant la cinématique de la prothèse dans les mouvements d'extension et de flexion ;
- les figures 10 à 15 sont des vues en plan du composant tibial correspondant, respectivement, aux figures 4 à 9.

D'une manière parfaitement connue, la prothèse du genou à glissement comprend, pour l'essentiel, un composant fémoral (1) et un composant tibial (T) comprenant un plateau mobile en polyéthylène (2) et une embase tibiale métallique (3). Le composant fémoral (1) présente une trochlée (1a) prolongée, de part et d'autre d'une échancrure (1b), par deux patins distaux condyliens (1c) et (1d) et deux patins postérieurs condyliens (le) et (1f). Le composant fémoral (1) est de forme générale anatomique et peut présenter une section interne de forme congruente à la forme anatomique de la tête fémorale ou bien, comme illustré aux figures des dessins, une série de pans coupés.

Le plateau tibial (2) est monté avec capacité de rotation et de translation guidée par rapport à l'embase tibiale (3). Dans ce but, le plateau mobile (2) présente, sensiblement dans sa partie médiane, une ouverture (2a) de forme générale oblongue et délimitant, d'une manière symétrique, de part et d'autre, deux cupules congruentes (2b) et (2c) avec les patins condyliens (1c) et (1d), notamment en position d'extension et de début de flexion du cycle de marche, puis en position de flexion maximum, comme il sera indiqué dans la suite de la description. L'ouverture médiane (2a) coopère, par exemple, avec un axe pivot (3a) que présente l'embase tibiale (3).

Un plot (4) est engagé au travers de l'ouverture (2a) et centré dans l'axe pivot (3a). Une vis (5) ou autre moyen d'assemblage assure la fixation du plot (4) avec l'embase tibiale (3) en étant, par exemple, vissé dans une quille tibiale (3b).

D'une manière importante, l'axe pivot (3a) est asymétrique, en ce sens qu'il présente, du côté antérieur, une portée de plus grande hauteur (3a1) permettant de renforcer le plot (4) qui, avantageusement, est réalisé en polyétylène.

Dans l'exemple illustré, le plot (4) présente une embase circulaire (4a) apte à être centrée dans l'axe pivot (3a).

Le plot (4) présente une tête (4b) formant une zone d'appui profilée (4c) apte à coopérer avec un élément profilé faisant office de came (6) que présente une partie du composant fémoral (1). Plus particulièrement, l'élément profilé en came (6) est disposé transversalement entre les deux patins condyliens (1c) et (1d) au niveau de l'extrémité libre de l'échancrure intercondylienne (1b).

Comme il sera indiqué dans la suite de la description, au moment de l'analyse de la cinématique de la prothèse, l'élément profilé en came (6), en position d'extension, n'est pas en contact avec le plot (4) mais vient seulement en contact au-delà d'un certain degré de flexion pour provoquer, d'une manière concomitante, au fur et à mesure de la flexion, le déplacement antéropostérieur du plateau (2) par rapport à l'embase tibiale (3).

Le plateau tibial (2) est donc monté avec capacité de mouvements de rotation autour de l'axe pivot (3a) et de déplacement d'avant en arrière et latéralement, l'ouverture (2a) ayant une largeur supérieure au diamètre de l'axe pivot (3a).

On rappelle que le plot (4) est engagé dans un axe pivot (3a) asymétrique pour éviter tout contact accidentel du plateau (2) contre ledit plot central (4) également réalisé en plastique et, avantageusement, en polyéthylène.

La face antérieure (2d) du plateau (2) n'est pas échancrée à l'inverse de celle de l'embase tibiale (3), afin d'utiliser le maximum de surface en appui lorsqu'il est position postérieure sur ladite embase.

A noter que le plot (4) peut être réalisé en différentes hauteurs, en fonction des différentes épaisseurs possibles du plateau mobile (2).

Avantageusement, l'élément profilé en came (6) a une forme générale en diabolo. L'embase tibiale (3) est plane et métallique, et présente une surface d'appui et de glissement polie pour faciliter, notamment le déplacement antéropostérieur du plateau mobile (2) sous l'effet de la flexion du composant fémoral (1) par rapport au composant tibial (T).

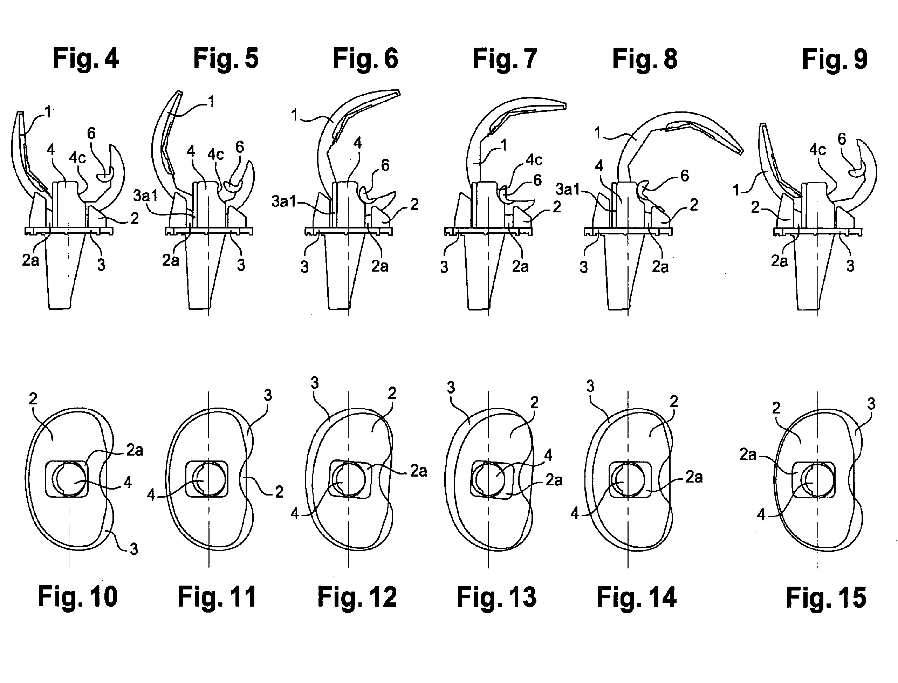

On renvoie aux figures 4 à 9 et aux figures 10 à 15 qui montrent la cinématique des composants de la prothèse selon l'invention permettant, notamment, d'assurer le déplacement antéropostérieur du plateau par rapport à l'embase en évitant tout mouvement relatif des patins condyliens (1c) et (1d) afin de diminuer, voire de supprimer, tout effet de délamination de la couche supérieure du polyéthylène. On note qu'en extension et en flexion minimale, le plateau ne se translate pas.

En mouvement d'extension (figures 4 à 10) jusqu'à une flexion d'environ 30° (figures 5 et 11), les patins condyliens (1c) et (1d) sont donc en parfaite congruence avec les cupules (2b) et (2c) du plateau mobile (2), ce qui permet d'avoir un genou hautement stabilisé.

Au fur et à mesure de la flexion du composant fémoral (1) par rapport au composant tibial (T), l'élément profilé en came (6) vient en butée avec la zone d'appui profilée (4c) du plot central (4) et se déplace par glissement le long de cette zone provoquant, d'une manière concomitante, la rotation externe, puis le déplacement postérieur du plateau mobile (2) par rapport à l'embase tibiale (3).

A la figure 6, l'angle de flexion de l'élément fémoral est d'environ 75°, l'élément profilé en came (6) est en appui avec la zone profilée (4c) du plot central (4), l'ouverture (2a) du plateau (2) n'étant pas encore en butée avec le plot (4). Le plateau pivote autour d'un point centré sur le milieu de la cupule interne (2c).

A la figure 7, la flexion est d'environ 90° correspondant à une position de butée de l'ouverture du plateau (2) avec le plot central (4). Le plateau conserve sa rotation.

Au-delà de cette flexion (figure 8), l'élément profilé en came (6) glisse le long de la zone d'appui profilée (4c) du plot central (4), toujours en butée dans le fond de l'échancrure du plateau mobile (2).

En position de flexion maximum (figures 9 et 15), les patins condyliens (le) et (If) sont en congruence avec le plateau (2). Il y a un mouvement de recul du plateau (2) par rapport à l'embase (3). Le plateau perd graduellement de sa rotation, puis se déplace parallèlement vers l'arrière.

Les avantages ressortent bien de la description, en particulier on souligne et on rappelle :
- la présence de deux variantes fonctionnelles de prothèse en un seul implant ;
- la limitation de flexion et la diminution de l'usure du polyéthylène ;les mouvements de translation / rotation des patins condyliens sur le plateau mobile, lors de mouvements de la marche notamment.

## Revendications

1. Prothèse totale du genou à plateau mobile (2) comprenant un composant fémoral (1) constitué d'une trochlée (1a) prolongée, de part et d'autre d'une échancrure (1b), par deux patins condyliens (1c) - (le) et (1d) - (1f) coopérant, avec capacité de flexion et de rotation, avec le plateau mobile (2) monté avec capacité de déplacement antéropostérieur limité par rapport à une embase (3) que présente un composant tibial (T), le composant fémoral (1) présente un élément profilé en came (6) disposé transversalement entre les deux patins condyliens (1c) et (1d), au niveau de l'extrémité libre de l'échancrure intercondylienne (1b), et apte à coopérer avec une zone d'appui profilée (4c) d'une manière correspondante que présente un plot (4) rendu solidaire de ladite embase, le plateau (2) présentant sensiblement dans sa partie médiane une ouverture (2a) de forme générale oblongue délimitant d'une manière symétrique deux cupules congruentes avec les patins condyliens, ledit élément profilé en came (6) coopérant avec la zone d'appui profilée (4c) en position de flexion à partir d'un angle d'environ 75 ° de manière à provoquer, d'une manière concomitante, le déplacement antéropostérieur du plateau (2) par rapport à l'embase (3), puis le recul limité dudit plateau (2) en position de flexion maximum du composant fémoral (1) par rapport au composant tibial (T), **caractérisée en ce que** ladite ouverture coopère avec un axe pivot (3a) de l'embase tibiale, ledit plateau étant monté avec capacité de mouvements de rotation autour dudit axe pivot (3a) et de déplacement d'avant en arrière et latéralement, l'ouverture (2a) ayant une largeur supérieure au diamètre de l'axe pivot (3a), le plot (4) étant engagé dans l'axe pivot (3a) et débordant de l'embase (3) au travers de l'ouverture (2a) du plateau.

2. Prothèse selon la revendication 1, **caractérisée en ce que** l'élément (6) profilé en came a une forme générale en diabolo.

3. Prothèse selon l'une des revendications 1 et 2, **caractérisée en ce que** le plot (6) est en polyéthylène et est rendu solidaire de l'embase (3) par une vis (5) engagée dans une quille tibiale (3b) que présente ladite embase (3).

4. Prothèse selon la revendication 1, **caractérisée en ce que** l'embase tibiale (3) est plane et métallique et présente une surface d'appui et de glissement polie.

5. Prothèse selon l'une quelconque des revendications 1 à 4, **caractérisée en ce que** le déplacement antéropostérieur du plateau (2) par rapport à l'embase (3), s'effectue sous l'effet de la coopération du profilé en came (6) avec le plot (4), depuis un angle de flexion d'environ 75° par rapport à l'axe du fémur jusqu'à un angle d'environ 125°.

## Patentansprüche

1. Totalprothese des Knies mit mobiler Platte (2), umfassend eine aus einer verlängerten Patella (1a) gebildete Oberschenkelkomponente (1), auf jeder Seite eine Aussparung (1b), die durch zwei Kondylenkufen (1c) - (1e) und (1d) - (1f) durch eine Flexions- und Rotationskapazität mit der mobilen Platte (2) zusammenwirken, welche mit einer Verschiebungskapazität von vorne nach hinten im Verhältnis zu einer Basis (3) montiert ist, die eine Schienbeinkomponente (T) aufweist, die Oberschenkelkomponente (1) weist ein profiliertes Element in Nockenform (6) auf, das quer zwischen den zwei Kondylenkufen (1c) und (1d) am freien Ende der Interkondylen-Aussparung (1b) angeordnet ist und geeignet ist, mit einem profilierten Stützbereich (4c) auf entsprechende Weise zusammenzuwirken, die ein fest mit der genannten Basis verbundenes Kontaktplättchen (4) aufweist, wobei die Platte (2) in ihrem mittleren Teil deutlich eine Öffnung (2a) in allgemeiner länglicher Form aufweist, die auf symmetrische Weise zwei kongruente Kapseln mit Kondylenkufen begrenzt, wobei das genannte profilierte Element in Nockenform (6) mit dem profilierten Stützbereich (4c) in Flexionsposition ab einem Winkel von rund 75° derart zusammenwirkt, so dass gleichzeitig die Verschiebung der Platte (2) von vorne nach hinten im Verhältnis zur Basis (3) und dann das begrenzte Zurückweichen der genannten Platte (2) in die maximale Flexionsposition der Oberschenkelkomponente (1) im Verhältnis zur Schienbeinkomponente (T) hervorgerufen wird, **dadurch gekennzeichnet, dass** die genannte Öffnung mit einer Drehachse (3a) der Schienbeinbasis zusammenwirkt, wobei die genannte Platte mit einer Rotationsbewegungskapazität rund um die genannte Drehachse (3a) und einer Verschiebungskapazität von vorne nach hinten und lateral montiert ist, wobei die Öffnung (2a) eine größere Breite hat als der Durchmesser der Drehachse (3a), wobei das Kontaktplättchen (4) in die Drehachse (3a) eingegriffen ist und von der Basis (3) durch die Öffnung (2a) der Platte übersteht.

2. Prothese gemäß Anspruch 1, **dadurch gekennzeichnet, dass** das profilierte Element in Nockenform (6) eine allgemeine Form einer Ziehrolle aufweist.

3. Prothese gemäß Anspruch 1 und 2, **dadurch gekennzeichnet, dass** das Kontaktplättchen (6) aus Polyethylen ist und mit der Basis (3) durch eine Schraube (5) fest verbunden wird, die in einen Schienbeinkegel (3b) eingreift, den die genannte Basis (3) aufweist.

4. Prothese gemäß Anspruch 1, **dadurch gekennzeichnet, dass** die Schienbeinbasis (3) eben und metallisch ist und eine polierte Stütz- und Gleitfläche aufweist.

5. Prothese gemäß Anspruch 1 bis 4, **dadurch gekennzeichnet, dass** die Verschiebung der Platte (2) von vorne nach hinten im Verhältnis zur Basis (3) unter der Wirkung des Zusammenwirkens des Profils in Nockenform (6) mit dem Kontaktplättchen (4) von einem Flexionswinkel von rund 75° im Verhältnis zur Achse des Oberschenkelknochens bis zu einem Winkel von rund 125° erfolgt.

## Claims

1. Total knee prosthesis with a movable plate (2), which comprises a femoral component (1) made up of a trochlea (1a) extended on either side of a notch (1b) by two condylar pads (1c) - (1e) and (1d) - (If). These pads, which can bend and rotate, work in conjunction with the movable plate (2), which is capable of limited anteroposterior displacement with respect to a base (3), which is part of a tibial component (T). The femoral component (1) has a cam profile element (6) that is placed crosswise between the two condylar pads (1c) and (1d), at the free end of the inter-condylar notch (1b), and that is capable of working in conjunction with a supporting area (4c) that is profiled accordingly. This supporting area is part of a post (4) that is securely attached to the aforementioned base. This prosthesis is **characterised by** the fact that the plate (2) has, roughly in its middle, an opening (2a) that is generally oblong in shape, which delimits two symmetric cavities that are the same size as the condylar pads. The aforementioned opening works in conjunction with a pivot axis (3a) on the tibial base; the aforementioned plate is designed so that it can rotate about the aforementioned pivot axis (3a), and move from front to back as well as laterally; the breadth of the opening (2a) is greater than the diameter of the pivot axis (3a); the aforementioned cam profile element (6) works in conjunction with the profiled supporting area (4c), in a flexed position at an angle greater than around 75°, so as to concomitantly result in the anteroposterior displacement of the plate (2) with respect to the base (3), followed by the limited recoil of the aforementioned plate (2) in the femoral component's (1) maximally flexed position with respect to the tibial component (T); the post (4) is inserted in the pivot axis (3a) and comes out of the base (3) after passing through the opening (2a) of the plate.

2. Prosthesis according to claim 1, **characterised by** the fact that the cam profile element (6) has the general shape of a diabolo.

3. Prosthesis according to one of claims 1 and 2, **characterised by** the fact that the post (6) is made of polyethylene and is securely attached to the base (3) with a screw (5) that is inserted in a tibial keel (3b) on the aforementioned base (3).

4. Prosthesis according to claim 1, **characterised by** the fact that the tibial base (3) is flat and metallic, with a polished sliding and supporting surface.

5. Prosthesis according to any one of claims 1 to 4, **characterised by** the fact that the anteroposterior displacement of the plate (2) with respect to the base (3) occurs due to the cam profile element (6) working in conjunction with the post (4), at a flexing angle ranging from around 75° to 125° with respect to the axis of the femur.
